# EUROPEAN PATENT APPLICATION

(11) **EP 4 205 629 A1**
(43) Date of publication of application: **05.07.2023**
(21) Application number: 22209370.0
(22) Date of filing: 24.11.2022
(51) Int. Cl.: A61B 1/00, A61B 1/12

(54) **ALBARRAN AND PULL-WIRE CARRIER FOR AN ALBARRAN**

(30) Priority: 04.01.2022 US 202263296320 P
(71) Applicant: Olympus Winter & Ibe GmbH, 22045 Hamburg (DE)
(72) Inventor: Schmuck, Irina, 25746 Heide (DE); Schulz, Kevin Alexander, 22889 Wilstedt (DE); Schröder, Bastian, 22589 Hamburg (DE); RÜHS, Andreas, 22926 Ahrensburg (DE)
(74) Representative: Hoener, Matthias

(57) **Abstract**

The invention provides an albarran (10) and a pull-wire carrier (25) that are particularly efficient to use and to manufacture and reprocess. This is achieved in that a pull-wire carrier (25) for an albarran (10) has a through hole (26) for a tubular shaft (12). This pull-wire carrier(25) can be mounted in a driving body (19) of the albarran (10), whereby pull wires (17) can be braced on the pull-wire carrier (25) for actuating a lever (15) of the albarran (10). This pull-wire carrier(25) is characterised by a receptacle (28) into which a cover element (29) can be inserted, the cover element (29) being detachably connectable to the pull-wire carrier (25). The at least one pull wire (17) can be fixed between the pull-wire carrier (25) and the cover element (29).

## Description

The invention relates to a pull-wire carrier according to claim 1. Furthermore, the invention relates to an Albarran according to claim 12.

Albarrans are used to support operations or treatments with surgical instruments such as endoscopes, resectoscopes, cystoscopes or similar. An Albarran can be used, for example, to deflect a flexible forceps inside a patient in a targeted and controlled manner. For this purpose, the Albarran, just like an endoscope for example, has a rod or tube-like shaft, which is to be guided into the patient's body with a distal end. At a proximal end of the Albarran, outside the patient, the shaft is connected to a main body. Via this main body, further instruments or tools, such as optics, wires or the like, can be guided through the shaft into the patient via various openings or ports.

At the distal end of the shaft, the Albarran has a lever, the so-called Albarran lever. This lever is designed to be movable and can be actuated or swivelled via a toggle on the main body. For this purpose, the lever is mechanically coupled with the toggle along the shaft. This mechanical coupling can be either a rod or a pull wire. Usually, the lever is connected to the toggle via two pull wires. It is conceivable that both pull wires serve equally to swing the lever back and forth or that different movements of the lever via the toggle can be effected via the two pull wires.

For reliable operation of the Albarran lever, the pull wires must be tensioned within the main body and there in a drive body with a pull-wire carrier. This means that the pull wires are screwed together under mechanical tension in known assembly procedures. For this purpose, the pull wires (or tension wires) are each fixed by a grub screw. In order for the grub screws to be able to tension the pull wire, the wire must be made deformable by means of annealing. This step is considered to be particularly time-consuming, as the wire already laid in the instrument has to be exposed to a sufficiently large amount of heat energy in the main body and then tensioned with the grub screw. Since this tensioning must be done from the side, i.e. transversely to a longitudinal axis of the shaft, this assembly step must be done before the toggle or a toggle axe is installed. The grub screws are screwed over the free openings for the toggles in the drive body. All in all, this assembly is very time-consuming, complicated and therefore prone to errors. If the main body of the Albarran does not allow such lateral access to the pull wire, this type of tensioning cannot be carried out. Furthermore, the material of the pull-wire carrier must be such that it can withstand both the heat input and the mechanical stress caused by the grub screws.

For reprocessing of the Albarran or the main body, it is essential that all components, especially those inside, can be rinsed, disinfected and sterilised and are arranged in such a way that no rinsing shadows or difficult-to-clean dead volumes are formed. These requirements are not met by the materials of the known systems alone. In addition, distances between moving components or between contact surfaces, such as the raw shaft and the pull-wire carrier, turn out to be impossible to clean. Without being able to achieve the necessary thoroughness in reprocessing, surgical instruments such as the Albarran described here and its components are not reusable. However, offering or using such complex instruments as *single-use instruments* is too cost-intensive.

In order to fulfil the aforementioned conditions, it is particularly important not to lose sight of the type of materials used and the dimensioning of the individual components. Especially in the case of mechanical components through which forces are to be exerted, care must be taken to ensure that the mechanical stability is sufficient to withstand even greater loads. Similarly, it must be taken into account that components may become defective, especially if they are misused. The consequences of such a defect for the treatment, but also for the patient, must be taken into account in the selection of materials or in the dimensioning of all components. Likewise, measures are required to react to possible defects of individual components.

The invention is based on the problem of creating an albarran and a pull-wire carrier that can be used particularly efficiently and that can be manufactured and reprocessed.

A solution to this problem is described by the features of claim 1. Accordingly, it is provided that a pull-wire carrier for an albarran has a through bore for a tubular shaft. This pull-wire carrier is mountable in a driving body of the albarran, whereby pull wires for actuating a lever of the albarran are braceable on the pull-wire carrier. This pull-wire carrier is characterised by a receptacle into which a cover element can be inserted, whereby the cover element can be detachably connected to the pull-wire carrier (or tension wire driver). The at least one pull wire can be fixed between the pull-wire carrier and the cover element. This fixing of the at least one pull wire by the cover element can be carried out from the proximal side of the pull-wire carrier and is therefore not limited by the design of the driving body. The pull wire can be fixed between the cover element and the pull-wire carrier in a simple, safe and thus reliable manner. This purely mechanical fixation makes the previously necessary heating or annealing of the wire superfluous.

Preferably, the invention further provides that the cover element can be clamped, pressed, glued and/or screwed in the receptacle of the pull-wire carrier. The aforementioned types of fixation can ensure that the at least one pull wire is fixed to the pull-wire carrier under the necessary mechanical tension. Both by clamping, pressing and screwing, the at least one pull wire can be fixed to the pull-wire carrier under defined conditions. This is particularly advantageous for a reproducible and thus reliable assembly of the Albarran.

In particular, the invention further provides that the cover element is placed in the receptacle with a predetermined pressure, preferably that the cover element is screwable with an end face with a defined torque against a receiving side of the receptacle of the pull wire carrier. In particular, the invention can provide that the distal end of the at least one pull wire can be placed or bent against the receiving side of the receptacle of the pull-wire carrier and can be clamped between the receiving side of the receptacle of the pull-wire carrier and the end face of the cover element, whereby a slip clutch of the pull wire is formed between the receiving side and the front face. This slip clutch of the pull wire causes the pull wire to slip out of the pull-wire carrier or out of the connection between the pull-wire carrier and the cover element in the event of misuse or a mechanical defect. This can prevent, for example, the lever or the axle of the lever from breaking or a pull wire from breaking. Especially due to a mechanical damage of the lever and/or the axle, unforeseeable complications can occur during the treatment. By using the slipping clutch described above, such mechanical damage can be counteracted in a defined manner. By applying a predetermined torque for fastening the wire ends in the pull-wire carrier, this slip clutch can be adjusted with a high degree of accuracy.

The invention may further provide that one or two holes for receiving one or two pull wires are arranged in the pull wire carrier, the holes extending from a distal side of the pull wire carrier to the receiving side of the pull wire receiver. Through these holes, the pull wires can be guided in a defined as well as safe manner to the proximal end of the pull-wire carrier, where they are fixed. The distal openings of these holes can be slightly enlarged to reduce the sliding resistance and to facilitate the threading of the wires into the holes. It is conceivable that these holes have a non-zero angle with respect to a longitudinal axis of the Albarran, in order to optimise the feeding of the wires to the receiving side of the pull-wire carrier. Equally, however, it is envisaged that the holes are aligned parallel to the longitudinal axis of the albarran.

A further advantageous embodiment of the invention may provide that the, in particular circular, through-bore in the pull-wire carrier for receiving a tube has one, two or more clearances on an inner wall. These clearances preferably extend parallel to a longitudinal axis of the Albarran over the entire length of the through hole. These clearances behave advantageously during purging/flushing of the albarran or the main body. When purging/flushing around as well as through the pull-wire carrier, the purging/flushing liquid can flow through this clearance along the inner wall of the through-hole and the outer surface of the tube. By this rinsing of a particularly heated rinsing liquid, a high degree of cleaning or sterilisation can be achieved, whereby the reprocessing of the instrument becomes possible.

According to the invention, it can be provided that the one, two or more clearances account for 10 % to 40 %, preferably 20 %, of the area of the inner wall of the through hole of the pull-wire carrier. The larger the sum of the areas of the clearance, the greater the achievable cleaning performance. However, an increasing area of the clearance also reduces the quality of the guidance of the shaft within the pull-wire carrier. Therefore, the mentioned area ratio has proven to be particularly advantageous for the use of the present instrument.

Furthermore, the invention may provide that a diameter of the through hole is larger than a diameter of a tube or shaft receivable in the through hole, wherein an annular gap is formed between the inner wall of the through hole and the tube. Here, too, the annular gap should be kept as small as possible so as not to adversely affect the necessary guidance of the tube within the pull-wire carrier. Similarly, the pull-wire carrier or the through-hole can be flushed more thoroughly if the annular gap is larger.

Further, it may be provided by the invention that a cross-section of the pull-wire carrier is wedge-shaped or trapezoidal, the cross-section tapering towards an upper end of the pull-wire carrier. Due to this shape of the upper section of the pull-wire carrier, a purge/flushing flow that hits the pull-wire carrier from above is divided into two partial flows that flush around the pull-wire carrier on different sides of the latter. These two flushing currents can each flow through a clearing of the pull-wire carrier. By splitting the flushing flow, both the outside and the through hole of the pull-wire carrier can be cleaned particularly efficiently. Through this flushing around and through, all flushing shadows within the main body of the Albarran can be reached. Such thorough cleaning enables the instrument to be reprocessed after treatment. Furthermore, it is conceivable that the pull-wire carrier has further shapes in order to positively influence the rinsing. In particular, round shapes are to be preferred in order to form the lowest possible flow resistance around the pull-wire carrier.

Preferably, it is conceivable that the width of the pull-wire carrier corresponds at most to twice the diameter of the through hole. This reduced width of the pull-wire carrier makes it possible to achieve a particularly narrow shape that can be easily washed around. In addition, material and thus costs can be saved by reducing the size.

A particularly advantageous embodiment provides for the pull-wire carrier to be made of a plastic, especially PEEK. PEEK in particular has proven to be particularly suitable for withstanding the mechanical requirements. In addition, PEEK can be cleaned particularly thoroughly. The sliding resistance between the pull-wire carrier and the metallic shaft has also proven to be sufficiently low when PEEK is used.

An albarran for solving said problem has the features of claim 12. Accordingly, it is provided that the Albarran has a tube-like shaft, at the distal end of which an Albarran lever is arranged and at the proximal end of which a main body with a driving body is arranged. The Albarran lever is movably connected to a toggle on the driving body via at least one, preferably two, pull wires. The pull wires can be fixed at a proximal end in the driving body to a pull wire carrier. The pull-wire carrier is designed according to at least one of claims 1 to 11.

A particularly advantageous embodiment of the Albarran can provide that a flushing connection is arranged on the driving body, whereby the flushing connection encloses an acute angle with the shaft pointing in the distal direction of the Albarran. This orientation of the flushing port directs the flushing fluid within the power unit in the proximal direction of the albarran. As soon as the flushing liquid flows through the flushing connection, which may in particular be a Lüer lock, into the interior of the driving body, it is split into two partial flows by the pull-wire carrier before it leaves the driving body again, inter alia by being cleared in the pull-wire carrier. The proximally inclined arrangement of the rinsing connection allows the rinsing liquid to flow completely through the interior space of the power unit or the main body. The residues loosened by the flushing liquid, in particular warm flushing liquid, leave the driving body in the distal direction or can be led away through one or two lateral outlets of the Albarran.

Another embodiment may provide that an axle of the Albarran lever is reinforced. This reinforcement may involve the use of a particularly stable material and/or an increase in the diameter of the axle. By forming the slip coupling described above and reinforcing the axle, a "predetermined breaking point" can thus be established, with which the risk of injuring the patient during the operation due to a defective lever is minimised.

A preferred embodiment of the present invention is explained in more detail below with reference to the drawing. In this show:
- Fig. 1: a perspective view of an albarran,
- Fig. 2: a side view of a distal end of the albarran according to Fig. 1,
- Fig. 3: a side view of a pull-wire carrier,
- Fig. 4: a view of a proximal end of the pull-wire carrier according to Fig. 3,
- Fig. 5: a view of a proximal end of the pull-wire carrier according to Fig. 3,
- Fig. 6: a view of a proximal end of the albarran according to Fig. 1,
- Fig. 7: a side view of a proximal end of the albarran according to Fig. 1, and
- Fig. 8: a view of the proximal end of the albarran according to Fig. 1.

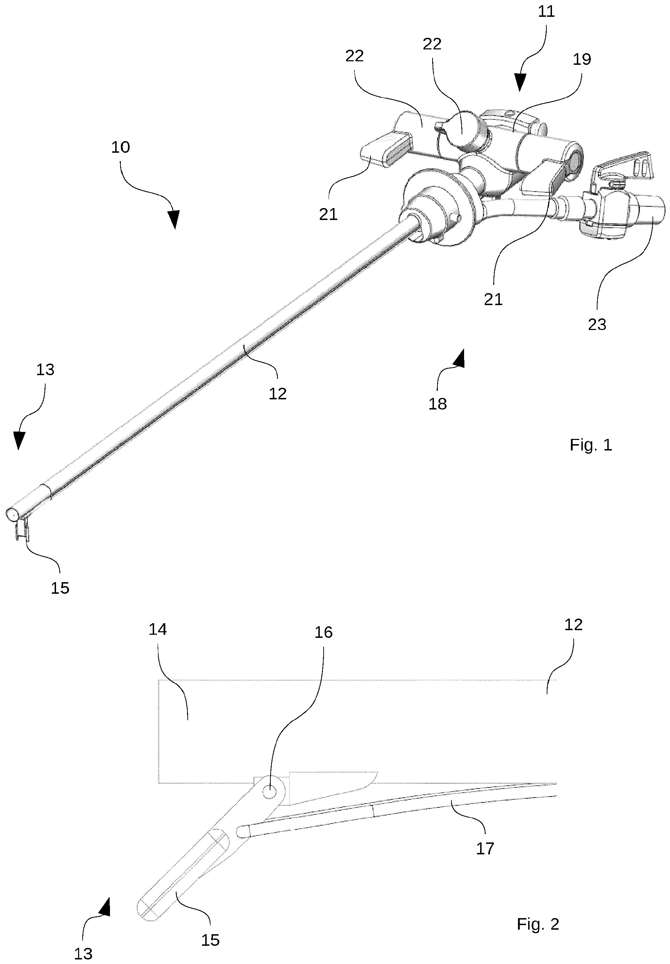

Fig. 1 shows a possible embodiment of an albarran 10. It should be expressly noted that the scope of protection of the present patent application is not intended to be limited to this embodiment. Rather, it is intended that further embodiments are also covered by the scope of protection.

The albarran 10 essentially consists of a main body 11 and a shaft 12 mounted on the main body 11. The shaft 12 is tubular and is guided into the body of the person for the treatment of persons with a distal end 13 in front. Various medical or surgical instruments can be passed through the shaft 12. An albarran lever or lever 15 is movably arranged on an outer wall 14 of the distal end 13 of the shaft 12. This lever 15 serves the surgeon during treatment as an aid for carrying out further treatment steps. The lever 15 is mounted so that it can be swivelled about an axis 16 (Fig. 2). To move the lever 15, it can be actuated via pull wires 17. In the example shown here, two pull wires 17 are assigned to the lever 15. However, embodiments are also conceivable in which the lever 15 has only one pull wire 17. Furthermore, embodiments are conceivable in which the pull wire 17 is designed as a rod.

The pull wires 17 run parallel to the shaft 12 from the lever 15 to the proximal end 18 of the albarran 10, passing through the main body 11 and being secured in a driving body 19 of the main body 11. To actuate the lever 15, the actuator body 19 has a toggle 20 with two actuating means 21. By actuating or rotating these actuating means 21, the pull wires 17 are moved forward or backward along the shaft 12, thereby performing a pivoting movement of the lever 15 about the axis 16.

In order to be able to clean the albarran 10 and in particular the main body 11, a purge/flushing connection 22 is arranged on the drive body 19. This purge/flushing connection 22 forms an acute angle with the shaft 12 and is thus inclined towards the distal end 13. Through this purge/flushing connection 22, a flushing liquid can be conveyed into the drive body 19. The fluid can leave the main body 11 again, for example at a distal end, or be discharged or sucked out via two discharges 23. While the purge/flushing connection 22 can be equipped with a cover for closing the opening, the discharge 23 in the embodiment example of the albarran 10 shown here each have a valve. Via the discharges 23, the flushing liquid can be actively sucked out of the main body 11 by a pump. This allows the main body 11 or the entire albarran 10 to be rinsed in a particularly thorough manner.

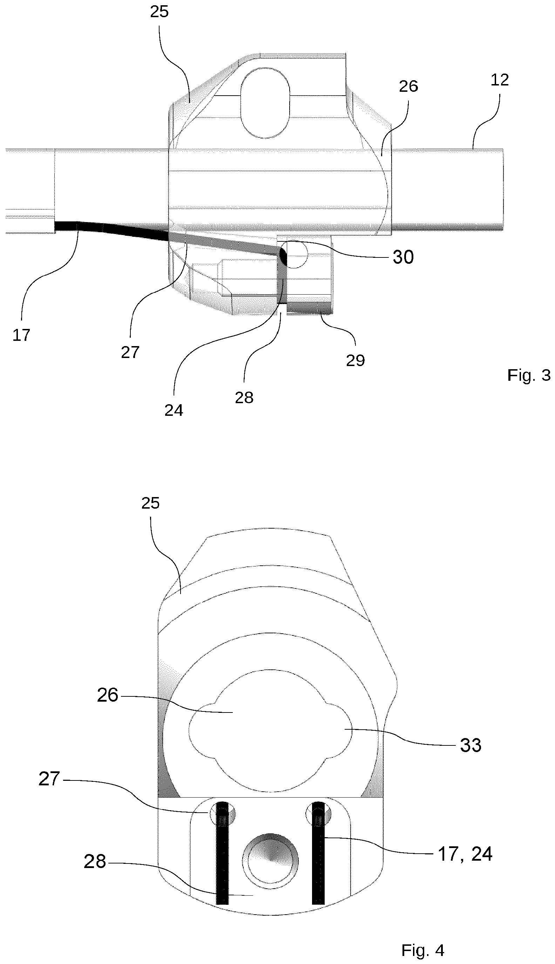

In order to be able to operate the lever 15 via the toggle 20, the proximal ends 24 of the pull wires 17 are attached to a pull-wire carrier 25 in the drive body 19. The pull-wire carrier 25 has a through hole 26 through which a tube or tube-like shaft 12 is passed. Thereby, the pull-wire carrier 25 is movably mounted on the shaft 12. For receiving the proximal ends 24 of the pull wires 17, the pull-wire carrier 25 has two bores 27 below the through hole 26. These bores 27 may be slightly inclined with respect to the through hole 26. The proximal ends 24 of the pull wires 17 protrude out of the pull-wire carrier 25 in a receptacle 28 of the pull-wire carrier 25. For sufficient locking of the pull wires 17, the proximal ends 24 are clamped in the receptacle 28 with a cover element 29. For this purpose, the proximal ends 24 are first bent against a receiving side 30 of the receptacle 28 so that the proximal ends 24 of the pull wires rest against the receiving side 30 (Fig. 4). In the following step, the cover element 29 is inserted into the receptacle 28 and screwed against the pull-wire carrier 25 with a screw 31. For this purpose, the cover element has a hole through which the screw 31 is passed. The pull-wire carrier 25 in turn has a bore with a corresponding internal thread in which the screw 31 can be screwed. The proximal ends 24 are clamped between an end face 32 of the cover element 29 and the receiving side 30 of the pull-wire carrier 25. By applying a predetermined torque to the screw 31, the proximal ends 24 of the pull wires 17 can be fixed in a very defined manner.

This defined fixation is particularly advantageous as it can be used as a slip clutch when using the albarran 10, especially the lever 15. There is a risk that in the event of a malfunction or defect, such a large force is transmitted to the lever 15 via the pull wires 17 that it breaks or causes other mechanical damage. As a consequence, the treatment is complicated and may result in avoidable trauma to the patient. By applying a predefined torque and by the way the proximal ends 24 of the pull wires 17 are fixed, before too much force can be applied, the pull wires 17 slip between the receiving side 30 and the front face 32, thus avoiding damage to the lever 15 or other serious mechanical defect of the albarran 10. Furthermore, the invention provides that the axle 16 of the lever 15 is reinforced or made of a particularly stable material in order to withstand higher mechanical forces.

The aforementioned manner in which the proximal ends 24 of the pull wires 17 are to be fastened to the pull-wire carrier 25 is also advantageous for the reason that the fastening can be carried out purely mechanically from the open proximal side of the main body 11. This is particularly easy and error-free to carry out. Re-tightening or re-fixing the pull wires 17 is also particularly advantageous in this embodiment.

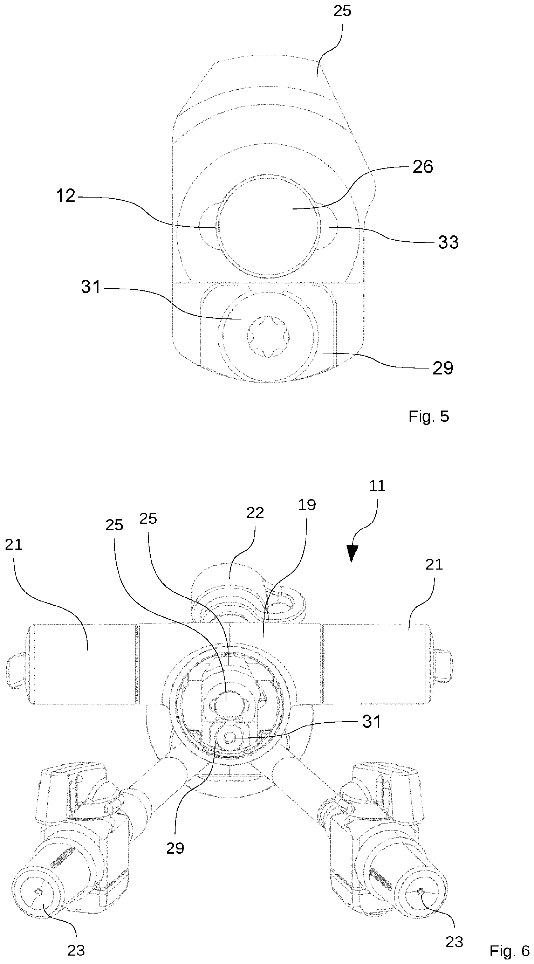

A particular challenge is the reprocessing of the albarran 10 or the main body 11. It is particularly challenging to reliably reach narrow spaces as well as dead volumes with the rinsing fluid. The invention provides that the through hole 26 of the pull-wire carrier 25 has at least one, two or more clearances 33 (Fig. 5). These clearances 33 are positioned parallel to the longitudinal axis of the through hole 26 on the inner wall of the pull-wire carrier 25 and substantially increase the area of the inner wall. The clearances 33 allow additional flushing fluid to pass through the pull-wire carrier 25, namely into the annular space between the shaft 12 and the through-hole 26. In this way, areas that were previously difficult to access can also be reached by the flushing fluid, in particular warm flushing fluid. Overall, the overall flushing behaviour of the main body 11 can be improved by the clearances 33.

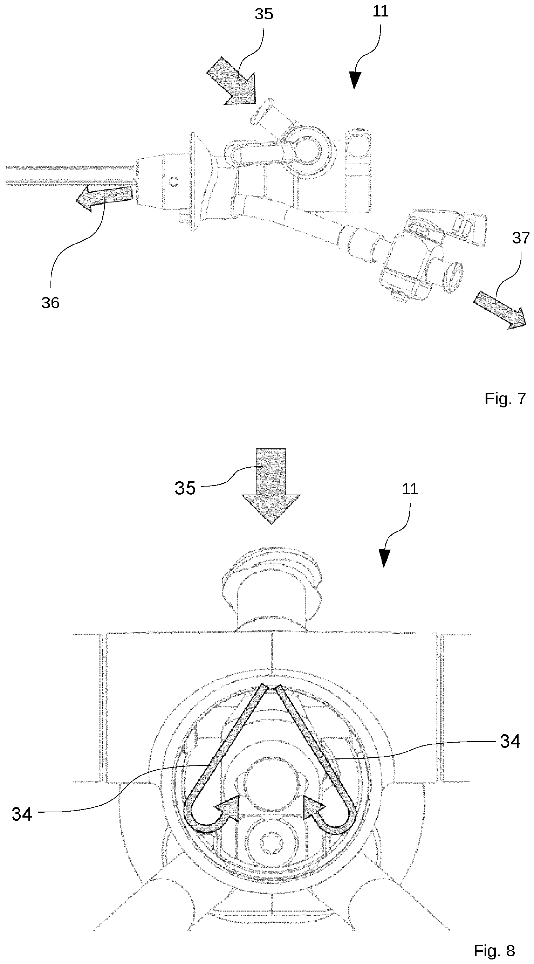

For a particularly efficient introduction of the rinsing liquid, the rinsing connection 22 is arranged directly above the pull-wire carrier 25 (Fig. 6). On diagonally opposite sides of the rinsing connection 22 are the two discharges 23, with which the contaminated rinsing liquid can be discharged quickly and efficiently. To ensure that the rinsing liquid is distributed particularly well within the body 19, the pull-wire carrier 25 is wedge-shaped or trapezoidal at one upper end. In Fig. 8, the arrows 34 illustrate how the rinsing liquid is guided through the rinsing connection into the drive body 19 according to the arrow 35 and is rinsed around the same due to the shape of the pull-wire carrier 25. Due to the inclined position of the flushing port 22, the flushing fluid is first directed in a proximal direction, where it is then directed into the clearances 33 and flushes around the space inside the pull-wire carrier 25 as well as outside the pull-wire carrier. As shown in Fig. 7, the flushing fluid can leave the main body 11 both in a distal direction 36 and through the discharges 23 in a proximal direction 37. It is conceivable that the flushing fluid is directed into the main body 11 via the flushing port 22 under an increased or variable or pulsed pressure. In particular, due to the reduced shape of the pull-wire carrier 25, a particularly efficient as well as comprehensive rinsing and flushing of the pull-wire carrier 25 and the driving body 19 can take place.

**List of reference signs:**

| | | | |
|---|---|---|---|
| 10 | Albarran | 36 | Distal direction |
| 11 | Main body | 37 | Proximal direction |
| 12 | Shaft | | |
| 13 | Distal end | | |
| 14 | Exterior wall | | |
| 15 | Lever | | |
| 16 | Axle | | |
| 17 | Pull wire | | |
| 18 | Proximal end | | |
| 19 | Driving body | | |
| 20 | Toggle | | |
| 21 | Actuating means | | |
| 22 | Purge connection | | |
| 23 | Discharge | | |
| 24 | Proximal end | | |
| 25 | Pull-wire driver | | |
| 26 | Through hole | | |
| 27 | Bore | | |
| 28 | Receptacle | | |
| 29 | Cover element | | |
| 30 | Receiving side | | |
| 31 | Screw | | |
| 32 | Front face | | |
| 33 | Clearance | | |
| 34 | Arrow | | |
| 35 | Arrow | | |

## Claims

1. Pull-wire carrier (25) for an albarran (10) with a through bore (26), wherein this pull-wire carrier (25) is mounted in a driving body (19) of the albarran (10) and wherein pull wires (17) for actuating a lever (15) of the albarran (10) can be braced on the pull-wire carrier (25), **characterised by a** receptacle (28) into which a cover element (29) can be placed, wherein the cover element (29) can be detachably connected to the pull-wire carrier (25) and at least one pull wire (17) can be fixed between the pull-wire carrier(25) and the cover element (29).

2. Pull-wire carrier (25) according to claim 1, **characterised in that** the cover element (29) can be clamped, pressed, glued and/or screwed in the receptacle (28) of the pull-wire carrier (25).

3. Pull-wire carrier (25) according to claim 1 or 2, **characterised in that** the cover element (29) is placed with a predetermined pressure in the receptacle (28), preferably that the cover element (29) can be screwed with an front face (32) with a defined torque against a receiving side (30) of the receptacle (28) of the pull-wire carrier (25).

4. Pull-wire carrier (25) according to claim 3, **characterized in that** the proximal end (24) of a pull wire (17) or the proximal ends (24) of two pull wires (17) can be placed against the receiving side (30) of the receptacle (28) of the pull-wire carrier (25) and can be clamped between the receiving side (30) of the receptacle (28) of the pull-wire carrier (25) and the front face (32) of the cover element (29), whereby a slip clutch of the pull wire (17) is formed between the receiving side (30) and the front face (32).

5. Pull-wire carrier (25) according to any one of the preceding claims, **characterized in that** one or two bores (27) for the receptacle (28) one or two pull wires (17) are arranged in the pull-wire carrier (25), the bores (27) extending from a distal side of the pull-wire carrier (25) to the receiving side (30) of the receptacle (28) of the pull-wire carrier (25).

6. Pull-wire carrier (25) for an albarran (10) with a through hole (26), wherein this pull-wire carrier (25) is mounted in a driving body (19) of the albarran (10) and wherein pull wires (17) for actuating a lever (15) of the albarran (10) can be braced on the pull-wire carrier (25), in particular according to one of the claims 1 to 5, **characterized in that** the, in particular circular, through hole (26) for receiving a tube has on an inner wall one, two or more clearances (33) which extend, preferably parallel to a longitudinal axis, over the entire length of the through hole (26).

7. Pull-wire carrier (25) according to claim 6, **characterized in that** the one, two or more clearances (33) occupy 10% to 40%, preferably 20%, of the area of the inner wall.

8. Pull-wire carrier (25) according to any one of the preceding claims, **characterised in that** a diameter of the through hole (26) is larger than a diameter of a tube receivable in the through hole (26), wherein an annular gap is formed between the inner wall of the through hole (26) and the tube.

9. Pull-wire carrier (25) according to any one of the preceding claims, **characterised in that** a cross-section of the pull-wire carrier (25) is wedge-shaped or trapezoidal, the cross-section tapering towards an upper end of the pull-wire carrier (25).

10. Pull-wire carrier (25) according to any one of the preceding claims, **characterised in that** a width of the pull-wire carrier (25) is at most twice the diameter of the through hole (26).

11. Pull-wire carrier (25) according to one of the previous claims, **characterised in that** the pull-wire carrier (25) is made of a plastic, preferably PEEK.

12. Albarran (10) with a tubular shaft (12), at the distal end (13) of which an albarran lever (15) is arranged and at the proximal end (18) of which a main body (11) with a driving body (19) is arranged, wherein the albarran lever (15) is movable via at least one, preferably two, pull wires (17) by means of a toggle (20) on the driving body (19) and a proximal end (18) of the at least one pull wire (17) is movable via a toggle (20) on the driving body (19), preferably two pull wires (17) by means of a toggle (20) on the drive body (19), and a proximal end (18) of the at least one pull wire (17) in the drive body (19) being fixable to a pull-wire carrier (25) according to one of claims 1 to 11.

13. Albarran (10) according to claim 12, **characterised in that** a purge connection (22) is arranged on the drive body (19), the purge connection (22) forming an acute angle with the shaft (12) pointing in the distal direction of the albarran (10).

14. Albarran (10) according to claim 12 or 13, **characterised in that** the purge connection (22) is arranged directly above the pull-wire carrier(25) on the drive body (19), so that a purge fluid flow meets the wedge-shaped or trapezoidal pull-wire carrier(25) and can be divided into two partial flows (34).

15. Albarran (10) according to any one of claims 12 to 14, **characterised in that** an axle (16) of the albarran lever (15) is reinforced.
